# EUROPEAN PATENT APPLICATION

(11) **EP 4 234 680 A1**
(43) Date of publication of application: **30.08.2023**
(21) Application number: 21882879.6
(22) Date of filing: 20.10.2021
(51) Int. Cl.: C12N 1/20, A23L 33/135, A61K 8/99, A61K 35/747, A61P 17/14, A61Q 7/00

(54) **FOOD PRODUCT, COSMETIC, QUASI-PHARMACEUTICAL PRODUCT, AND PHARMACEUTICAL PRODUCT CONTAINING LACTIC ACID BACTERIA AND HAVING HAIR-GENERATING/HAIR-GROWING EFFECT**

(30) Priority: 21.10.2020 JP 2020176970
(71) Applicant: NISSIN FOODS HOLDINGS CO., LTD., Yodogawa-ku Osaka-shi, Osaka 532-8524 (JP)
(72) Inventor: MORI, Ayaka, Osaka-shi Osaka 532-8524 (JP); SUNADA, Yosuke, Osaka-shi Osaka 532-8524 (JP); TANII, Yusuke, Osaka-shi Osaka 532-8524 (JP); UEHARA, Kazuya, Osaka-shi Osaka 532-8524 (JP); IGI, Akemi, Osaka-shi Osaka 532-8524 (JP)
(74) Representative: Mewburn Ellis LLP
(86) International application number: PCT/JP2021/038824
(87) International publication number: WO 2022/085738

(57) **Abstract**

An object of the present invention is to develop a food or drink product, a cosmetic product, a quasi-drug or a medicament confirmed to help hair growth or the like through the actual oral intake of the food or drink product, the quasi-drug or the medicament by a human, by preparing the food or drink product, the cosmetic product, the quasi-drug or the medicament using the lactic acid bacterium concerned. The present inventors have screened various lactic acid bacteria and first selected a lactic acid bacterium (*Lactobacillus plantarum* species) by utilizing the fact that the activation of follicle dermal papilla cells is absolutely essential for obtaining a hair regrowth, hair growth, or pilatory effect, and screening a plurality of lactic acid bacteria with the activation of follicle dermal papilla cells as an index. The present inventors have further completed a food or drink product, a cosmetic product, or the like having a high hair growth effect by preparing a food or drink product using the lactic acid bacterium, and carrying out clinical trials involving actual intake by humans (test subjects) to confirm its effectiveness.

## Description

### Technical Field

The present invention relates to a food or drink product, a cosmetic product, a quasi-drug or a medicament having a hair regrowth or hair growth effect on the human body, wherein the food or drink product, the cosmetic product, the quasi-drug or the medicament comprises a lactic acid bacterium of a *Lactobacillus plantarum* species, or a culture solution or a fermentation product thereof.

### Background Art

Hairs help protection of the head from external force and from light such as ultraviolet ray or visible light. Besides, hairs are a factor that largely influences the appearance of the head from a fashion or beauty standpoint.

Hairs have a hair cycle in which growth and defluxion are repeated with a given cycle. The hair cycle can also be divided into the anagen, the catagen and the telogen.

Meanwhile, hair loss may occur easily depending on the predisposition of each individual due to a problem such as aging. Coping strategies including medicaments, quasi-drugs, and cosmetic products, such as hair growth products, are used as methods that deal with such hair loss or hair thinning for the head.

Various earlier filed patent applications disclose such coping strategies for hair loss or hair thinning. In general, a compound, for example, minoxidil or finasteride, is often used.

In recent years, research on lactic acid bacteria aimed at health maintenance has been energetically made as a method other than use of such a compound. Methods aimed at hair growth, hair regrowth or hair loss suppression using lactic acid bacteria are also disclosed.

### Citation List

### Patent Literature

Patent Literature 1: Japanese Patent Laid-Open No. 2015-187171

Patent Literature 1 relates to a method using a specific lactic acid bacterium strain of a specific genus *Lactococcus.* However, experimental results disclosed therein have been obtained using follicle dermal papilla cells by the measurement of the amount of a growth factor FGF-7 or VEGF produced, which is secreted from follicle dermal papilla cells. It is pointed out that the results are not those obtained by actual administration or the like to humans.

### Summary of Invention

### Technical Problem

Accordingly, the present inventors have paid attention to a novel lactic acid bacterium. An object of the present invention is to develop a method for obtaining a hair growth effect or the like on the human body using the lactic acid bacterium.

Particularly, an object of the present invention is to develop a food or drink product, a quasi-drug or a medicament confirmed to help hair growth or the like through the actual oral intake of the food or drink product, the quasi-drug or the medicament by a human, by preparing the food or drink product, the quasi-drug or the medicament using the lactic acid bacterium.

Another object of the present invention is to develop a cosmetic product, a quasi-drug or a medicament confirmed to help hair growth or the like through the actual application of the cosmetic product, the quasi-drug or the medicament in the form of a lotion or the like to the scalp by a human, by preparing the cosmetic product, the quasi-drug or the medicament using the lactic acid bacterium.

### Solution to Problem

The present inventors have screened various lactic acid bacteria and first selected a lactic acid bacterium (*Lactobacillus plantarum* species) by utilizing the fact that the activation of follicle dermal papilla cells is absolutely essential for obtaining a hair regrowth, hair growth, or pilatory effect, and screening a plurality of lactic acid bacteria with the activation of follicle dermal papilla cells as an index.

The present inventors have further completed a food or drink product having a high hair growth effect by preparing a food or drink product using the lactic acid bacterium, and carrying out clinical trials involving actual intake by humans (test subjects) to confirm its effectiveness.

Specifically, the first aspect of the invention of the present application provides
"a food or drink product, a cosmetic product, a quasi-drug or a medicament comprising a lactic acid bacterium having a hair regrowth or hair growth effect in the human body, or a culture solution or a fermentation product thereof".

Next, the food or drink product comprising the lactic acid bacterium may have a name selected from the group consisting of names of refreshing sweets, a lactic acid bacterium-containing food product, a lactic acid bacteria beverage and a soft drink.

Specifically, the second aspect of the invention of the present application provides
"the food or drink product, the cosmetic product, the quasi-drug or the medicament comprising the lactic acid bacterium or the culture solution or the fermentation product thereof according to claim 1, wherein the food or drink product is any one selected from the group consisting of products named as refreshing sweets, a lactic acid bacterium-containing food product, a lactic acid bacteria beverage and a soft drink".

Next, the food or drink product comprising the lactic acid bacterium of the present invention may be in the shape of a solid tablet as one example.

Specifically, the third aspect of the invention of the present application provides
"the food or drink product, the cosmetic product, the quasi-drug or the medicament comprising the lactic acid bacterium or the culture solution or the fermentation product thereof according to claim 1 or 2, wherein the food or drink product, the quasi-drug or the medicament is a tablet".

Next, the lactic acid bacterium is preferably a lactic acid bacterium that belongs to a *Lactobacillus plantarum* species.

Specifically, the fourth aspect of the invention of the present application provides
"the food or drink product, the cosmetic product, the quasi-drug or the medicament according to any of claims 1 to 3, wherein the lactic acid bacterium or the culture solution or the fermentation product thereof belongs to a *Lactobacillus plantarum* species".

Next, the lactic acid bacterium is preferably a *Lactobacillus plantarum* N793 strain (NITE BP-03233). Specifically, the fifth aspect of the invention of the present application provides
"the food or drink product, the cosmetic product, the quasi-drug or the medicament according to any of claims 1 to 4, wherein the lactic acid bacterium or the culture solution or the fermentation product thereof is a lactic acid bacterium which is *a Lactobacillus plantarum* N793 strain (NITE BP-03233), or a culture solution or a fermentation product thereof".

Next, the present inventors have further completed a cosmetic product, a quasi-drug or a medicament having a high hair growth effect by preparing a lotion (liniment) using the lactic acid bacterium, and carrying out clinical trials involving actual application to the scalp by humans (test subjects) to confirm its effectiveness.

The cosmetic product, the quasi-drug or the medicament comprising the lactic acid bacterium of the present invention is preferably a liniment.

Specifically, the sixth aspect of the invention of the present application provides
"the food or drink product, the cosmetic product, the quasi-drug or the medicament comprising the lactic acid bacterium or the culture solution or the fermentation product thereof according to claim 1, wherein the cosmetic product, the quasi-drug or the medicament is a liniment".

Next, the cosmetic product, the quasi-drug or the medicament comprising the lactic acid bacterium of the present invention is preferably an ointment, a cream or a lotion among the liniments.

Specifically, the seventh aspect of the invention of the present application provides
"the food or drink product, the cosmetic product, the quasi-drug or the medicament according to claim 6, wherein the cosmetic product, the quasi-drug or the medicament is an ointment, a cream or a lotion".

Next, the lactic acid bacterium is preferably a lactic acid bacterium that belongs to a *Lactobacillus plantarum* species.

Specifically, the eighth aspect of the invention of the present application provides
"the food or drink product, the cosmetic product, the quasi-drug or the medicament according to claim 6 or 7, wherein the lactic acid bacterium or the culture solution or the fermentation product thereof belongs to a *Lactobacillus plantarum* species".

Next, the lactic acid bacterium is preferably a *Lactobacillus plantarum* N793 strain (NITE BP-03233) that belongs to a *Lactobacillus plantarum* species.

Specifically, the ninth aspect of the invention of the present application provides
"the food or drink product, the cosmetic product, the quasi-drug or the medicament according to any of claims 6 to 8, wherein the lactic acid bacterium or the culture solution or the fermentation product thereof is a lactic acid bacterium which is *a Lactobacillus plantarum* N793 strain (NITE BP-03233), or a culture solution or a fermentation product thereof".

### Advantageous Effects of Invention

Use of the food or drink product, the quasi-drug or the medicament comprising the lactic acid bacterium or the culture solution or the fermentation product thereof according to the present invention enables hair loss or hair thinning to be effortlessly suppressed in the human body by the oral administration thereof.

Use of the cosmetic product, the quasi-drug or the medicament comprising the lactic acid bacterium or the culture solution or the fermentation product thereof according to the present invention enables hair loss or hair thinning to be effortlessly suppressed in the human body by the application or the like thereof.

### Brief Description of Drawings

[Figure 1] Figure 1 is a diagram showing results of evaluating the ability to activate follicle dermal papilla cells in Examples.
[Figure 2] Figure 2 is a diagram showing hair analysis results about the total number of hairs in a clinical trial (tablet).
[Figure 3] Figure 3 is a diagram showing hair analysis results about a hair thickness in a clinical trial (tablet).
[Figure 4] Figure 4 is a diagram showing hair analysis results about the number of non-soft hairs in a clinical trial (tablet).
[Figure 5] Figure 5 is a diagram showing hair analysis results about a non-soft hair ratio in a clinical trial (tablet).
[Figure 6] Figure 6 is a diagram showing hair analysis results about the number of soft hairs in a clinical trial (tablet).
[Figure 7] Figure 7 is a diagram showing hair analysis results about a soft hair ratio in a clinical trial (tablet).
[Figure 8] Figure 8 is a diagram showing hair analysis results about hair loss in a clinical trial (tablet).
[Figure 9] Figure 9 is a diagram showing results about the perception of hair loss at the time of hair washing in a VAS questionnaire (tablet).
[Figure 10] Figure 10 is a diagram showing results about the perception of hair loss at the time of hair styling in a VAS questionnaire (tablet).
[Figure 11] Figure 11 is a diagram showing results about the perception of a lack of hair firmness or elasticity in a VAS questionnaire (tablet).
[Figure 12] Figure 12 is a diagram showing results about the perception of hair thinness in a VAS questionnaire (tablet).
[Figure 13] Figure 13 is a diagram showing results about the perception of a lack of hair strength in a VAS questionnaire (tablet).
[Figure 14] Figure 14 is a diagram showing results about the perception of a lack of hair volume in a VAS questionnaire (tablet).
[Figure 15] Figure 15 is a diagram showing results about the perception of noticeable hair sparseness at the head skin (scalp) in a VAS questionnaire (tablet).
[Figure 16] Figure 16 is a diagram showing results about the perception of noticeable hair sparseness at the top of the head, hairline or parting in a VAS questionnaire (tablet).
[Figure 17] Figure 17 is a diagram showing results about the realization of the extent of hair loss in a VAS questionnaire (tablet).
[Figure 18] Figure 18 is a diagram showing hair analysis results about the total number of hairs in a clinical trial (lotion).
[Figure 19] Figure 19 is a diagram showing hair analysis results about a hair thickness in a clinical trial (lotion).
[Figure 20] Figure 20 is a diagram showing hair analysis results about the number of non-soft hairs in a clinical trial (lotion).
[Figure 21] Figure 21 is a diagram showing hair analysis results about hair loss in a clinical trial (lotion).
[Figure 22] Figure 22 is a diagram showing results about the perception of hair loss at the time of hair washing in a VAS questionnaire (lotion).
[Figure 23] Figure 23 is a diagram showing results about the perception of a lack of hair firmness or elasticity in a VAS questionnaire (lotion).
[Figure 24] Figure 24 is a diagram showing results about the perception of hair thinness in a VAS questionnaire (lotion).
[Figure 25] Figure 25 is a diagram showing results about the perception of a lack of hair strength in a VAS questionnaire (lotion).
[Figure 26] Figure 26 is a diagram showing results about the perception of a lack of hair volume in a VAS questionnaire (lotion).
[Figure 27] Figure 27 is a diagram showing results about the perception of noticeable hair sparseness at the head skin (scalp) in a VAS questionnaire (lotion).
[Figure 28] Figure 28 is a diagram showing results about the perception of noticeable hair sparseness at the top of the head, hairline or parting in a VAS questionnaire (lotion).
[Figure 29] Figure 29 is a diagram showing results about the realization of reduction in the extent of hair loss in a VAS questionnaire (lotion).

### Description of Embodiments

Hereinafter, the contents of the present invention will be described with reference to Examples. However, the present invention is not limited by Examples given below.

The first aspect of the invention of the present application provides "a food or drink product, a quasi-drug or a medicament comprising a lactic acid bacterium having a hair regrowth or hair growth effect in the human body".

### Hair regrowth or hair growth effect

The hair regrowth or hair growth effect described in the present invention refers to an effect of increasing the number of hairs or increasing a hair thickness, an effect of reducing hair loss, or the like and is any one or more of these effects.

### Lactic acid bacterium

Hereinafter, Examples of the present invention will be described. The present invention is not limited by Examples given below.

### Preparation of lactic acid bacterium

One example of the lactic acid bacterium used in the present invention is preferably a lactic acid bacterium of the genus *Lactobacillus.* More specifically, a lactic acid bacterium of a *Lactobacillus plantarum* species is preferred.

In the present invention, any lactic acid bacterium of a *Lactobacillus plantarum* species is possible. Among such lactic acid bacteria of *Lactobacillus plantarum* species, particularly, the following strain is preferably used.

### 1. Lactobacillus plantarum N793 strain (NITE BP-03233)

The lactic acid bacterium of the present invention is *Lactobacillus plantarum.* The lactic acid bacterium that belongs to *Lactobacillus plantarum* is, particularly, a *Lactobacillus plantarum* N793 strain (NITE BP-03233). The symbol N793 described in the present invention is a number given to the bacterial strain by Nissin Foods Holdings Co., Ltd. on their own accord. This *Lactobacillus plantarum* N793 strain has been discovered by the present inventors for the first time.

More specifically, this *Lactobacillus plantarum* N793 strain has been identified as a lactic acid bacterium that belongs to *Lactobacillus plantarum* subsp. *plantarum.*

The *Lactobacillus plantarum* N793 strain of the present invention was deposited as NITE BP-03233 with Patent Microorganisms Depositary, National Institute of Technology and Evaluation on June 18, 2020. The mycological properties of the *Lactobacillus plantarum* N793 strain of the present invention are as given in Tables 1 and 2. These mycological properties are based on a method described in Bergey's manual of systematic bacteriology Vol. 2 (1986). Table 1 shows the shape, etc. of the bacterial strain. Table 2 shows results of testing sugar assimilation using Api 50CH and Api CHL (manufactured by bioMerieux Japan Ltd.). In Table 2, "+" represents positive, and "-" represents negative.

**[Table 1]**

| Test item | | *Lactobacillus plantarum* N793 strain |
|---|---|---|
| Culture temperature | | 30°C |
| Cell morphology | | Rod (0.6-0.7×2.0-3.0 µm) |
| Gram stainabilitv | | + |
| Presence or absence of spore | | - |
| Motility | | - |
| Colony morphology | Medium | MRS agar medium |
| | Culture time | 24 hours |
| | Diameter | 2-3 mm |
| | Color | Milky white |
| | Shape | Round |
| | Raised state | Lens-like |
| | Perimeter | Entire margin |
| | Surface shape, etc. | Smooth |
| | Transparency | Opaque |
| | Viscosity | Butter-like |
| Growing temperature test | 37°C | + |
| | 45°C | + |
| Catalase reaction | | - |
| Oxidase reaction | | - |
| Acid/gas production from glucose | | +/- |
| O/F test (oxidation/fermentation) | | +/- |

| | | |
|---|---|---|
| +: positive, -: negative | | |

**[Table 2]**

| | Sugar substrate | result | | Sugar substrate | result |
|---|---|---|---|---|---|
| 0 | Control | - | 25 | Esuculin ferric citrate | + |
| 1 | Glycerol | - | 26 | Salicin | + |
| 2 | Erythritol | - | 27 | D-Cellobiose | + |
| 3 | D-Arabinose | - | 28 | D-Maltose | + |
| 4 | L-Arabinose | + | 29 | D-Lactose | + |
| 5 | D-Ribose | + | 30 | D-Melibiose | + |
| 6 | D-Xylose | - | 31 | D-Sucrose | + |
| 7 | L-Xylose | - | 32 | D-Trehaloses | + |
| 8 | D-Adonitol | - | 33 | Inulin | - |
| 9 | Methyl-βD-Xylopyranoside | - | 34 | D-Melezitose | + |
| 10 | D-Galactose | + | 35 | D-Raffmose | + |
| 11 | D-Glucose | + | 36 | Starch | - |
| 12 | D-Fructose | + | 37 | Glycogen | - |
| 13 | D-Mannose | + | 38 | Xylitol | - |
| 14 | L-Sorbose | - | 39 | Gentiobiose | + |
| 15 | L-Rhamnose | - | 40 | D-Turanose | + |
| 16 | Dulcitol | - | 41 | D-Lyxose | - |
| 17 | Inositol | - | 42 | D-Tagatose | - |
| 18 | D-Mannnitol | + | 43 | D-Fucose | - |
| 19 | D-Sorbitol | + | 44 | L-Fucose | - |
| 20 | Methy1-αD-Mannopyranosede | + | 45 | D-Arabitol | - |
| 21 | Methyl-αD-Glucopyranoside | - | 46 | L-Arabitol | - |
| 22 | N-Acetyl-Glucosamine | + | 47 | Gluconate | + |
| 23 | Amygdalin | + | 48 | 2-keto-Gluconate | - |
| 24 | Arubutin | + | 49 | 5-keto-Gluconate | - |

### Lactic acid bacterium powder or culture solution or fermentation product thereof

The lactic acid bacterium of the present invention can be used in various forms. For example, a freeze-dried type of the bacterial cells or a product obtained by enzymatically treating and freeze-drying the bacterial cells can be used as a lactic acid bacterium powder.

In addition to the lactic acid bacterium powder, a culture solution of the lactic acid bacterium of the present invention can also be used. Specifically, any culture solution containing the bacterial cells, culture supernatant, or the like can be used. The bacterial cells, etc. described above may be subjected, if necessary, to centrifugation or enzymatic treatment with protease, lipase, or the like, as a matter of course.

In addition to the lactic acid bacterium powder or the culture solution thereof, a fermentation product obtained using the lactic acid bacterium may be used. That is, a fermentation product obtained by purifying a compound, a peptide, or the like produced during a fermentation process from the lactic acid bacterium can also be used. Specific examples thereof include equol and GABA.

### Food or drink product

The lactic acid bacterium powder of the present invention or the culture solution or the fermentation product thereof can be contained in a food or drink product for use.

First, the food or drink product of the present invention can be in the form of a formulation of the lactic acid bacterium powder or the culture solution or the fermentation product thereof supplemented, if necessary, with an appropriate carrier and additive. Specifically, various forms such as powders, granules, capsules, tablets, dusts, coated tablets, liquids, syrups, and juices are possible.

The food or drink product comprising the lactic acid bacterium powder of the present invention or the culture solution or the fermentation product thereof can contain the bacterial cells of the present invention or the culture solution or the fermentation product thereof as an active ingredient and may further contain an additional component, as a matter of course.

Specific examples thereof include sugars (starch, dextrin, polysaccharides, monosaccharides, disaccharides, etc.), proteins, various amino acids, lipids, moisture, vitamins, minerals, flavors, and various food additives.

A beverage comprising the lactic acid bacterium of the present invention is also possible. Examples thereof include fermented milk containing the lactic acid bacterium and lactic acid bacteria beverages containing the lactic acid bacterium. Although the existing Ministerial Ordinance Concerning Compositional Standards, etc. for Milk and Milk Products does not particularly define a lactic acid bacterial count as compositional standards, the bacterial count is preferably 1.0 × 10⁷ cfu/ml or more for fermented milk (having a nonfat milk solid content of 8.0% or more) or a lactic acid bacteria beverage (having a nonfat milk solid content of 3.0% or more) and 1.0 × 10⁶ cfu/ml or more for a lactic acid bacteria beverage (having a nonfat milk solid content of less than 3.0%). The bacterial count described above can be achieved through growth in a fermented liquid such as milk or growth in the form of a final product. The lactic acid bacterium of the present invention may be used in dairy products such as butter, processed egg products such as mayonnaise, sweet baked goods such as butter cake, and the like, in addition to the fermented milk containing the lactic acid bacterium and the lactic acid bacteria beverages containing the lactic acid bacterium. Furthermore, the lactic acid bacterium of the present invention can also be suitably used in processed food products such as instant noodles and cookies.

The lactic acid bacterium of the present invention or the culture solution or the fermentation product thereof may be contained in foods for specified health use, nutritional supplements, foods with functional claims, and the like, in addition to the general food products or beverages, and this is also useful.

Examples of the name of the food or drink product according to the present invention include, but are not particularly limited to, refreshing sweets, lactic acid bacterium-containing food products, lactic acid bacteria beverages and soft drinks. However, the name is not limited thereto, as a matter of course.

### Quasi-drug, medicament (oral intake type)

The lactic acid bacterium powder of the present invention or the culture solution or the fermentation product thereof can also be contained, for use, in a quasi-drug or a medicament for oral intake, in addition to the food products. Specific examples thereof include forms that permit oral intake, such as drinkable preparations and tablet preparations.

That is, the lactic acid bacterium of the present invention or the culture solution or the fermentation product thereof is prepared into an extract or a powder by concentration, separation, or the like and can then be used as a quasi-drug or a medicament containing the component as an active ingredient. For example, the extract may be packed in a bottle or the like, or a dry powder of a fermented liquid may be granulated or encapsulated using an excipient and the like, tableted, and provided in the form of a tablet.

The quasi-drug or the medicament can contain the lactic acid bacterium powder of the present invention or the culture solution or the fermentation product thereof as an active ingredient and may further contain other components (e.g., sugars, proteins, amino acids, lipids, moisture, vitamins, and minerals), in addition to the active ingredient, as a matter of course.

### Cosmetic product, quasi-drug or medicament (liniment, etc.)

The lactic acid bacterium powder of the present invention or the culture solution or the fermentation product thereof can be contained in a cosmetic product, a quasi-drug or a medicament for use. Specifically, a liniment having a trade name of a pilatory product, a hair growth product, a hair regrowth products, or the like can be used. Specific examples thereof include forms such as ointments, creams, hair lotions, hair tonics, shampoos, and rinses.

Specifically, the lactic acid bacterium powder of the present invention or the culture solution or the fermentation product thereof is prepared into an extract or a powder by concentration, separation, or the like. Then, this extract or powder is diluted with or suspended in a predetermined solution, for example, and can be used as a cosmetic product, a quasi-drug or a medicament containing the component as an active ingredient.

In this context, for example, an ointment may be prepared by mixing a fat and oil base with the lactic acid bacterium powder or a suspension thereof. Also, a cream may be prepared by mixing an oil-in-water (O/W) or water-in-oil (W/O) liquid substance therewith. Further, a lotion includes a lotion in an emulsion form or a lotion in a suspension form. The emulsion form is an emulsified product, and the suspension form is a lotion containing an alcohol or water as a main agent.

The cosmetic product, the quasi-drug or the medicament can contain the bacterial cells of the present invention or the culture solution or the fermentation product thereof as an active ingredient and may further contain other components (e.g., sugars, proteins, amino acids, lipids, moisture, vitamins, and minerals), in addition to the active ingredient, as a matter of course.

### Examples

### 1. Screening of lactic acid bacterium

The lactic acid bacterium for use in the food or drink product, the quasi-drug or the medicament containing the lactic acid bacterium powder of the present invention or the culture solution or the fermentation product thereof was screened for as follows.

The hair cycle refers to a cycle in which hairs repeat three phases: the anagen, the catagen and the telogen. The longest period in the hair cycle is the anagen, which is usually on the order of 3 years to 6 years.

The anagen is maintained by growth factors such as keratinocyte growth factor, insulin-like growth factor 1, and vascular endothelial growth factor. These growth factors are produced from follicle dermal papilla cells residing deep in the hair root. The keratinocyte growth factor secreted from follicle dermal papilla cells is considered as a growth factor having the most direct action on hairs because this growth factor is known to act directly on hair matrix cells, which generate hairs, to promote the growth and division of the hair matrix cells. For these reasons, the follicle dermal papilla cells are cells necessary for hair regrowth. If follicle dermal papilla cells can be activated, this presumably leads to hair regrowth. Hence, various lactic acid bacteria were evaluated for their ability to activate follicle dermal papilla cells.

The bacterial species of the tested lactic acid bacteria were *Lactobacillus plantarum* (3 species), *Lactobacillus casei*/*paracasei* (3 species), *Lactobacillus rhamnosus* (3 species), *Lactobacillus fermentum* (3 species), *Lactobacillus reuteri* (3 species), and *Lactobacillus lactis* (3 species).

### 2. Follicle dermal papilla cell activation test

### <Preparation of test sample (lactic acid bacterium powder suspension)>

Each lactic acid bacterium was cultured at 37°C for 24 hours in MRS medium (manufactured by Becton, Dickinson and Company) shown in Table 3. Subsequently, the grown bacterial cells were harvested using a centrifuge. The harvested bacterial cells were washed with sterile water and harvested with the centrifuge. Washing and harvest were repetitively performed three times. After heat sterilization at 95°C for 15 minutes, the bacterial cells were harvested again with the centrifuge. The harvested bacterial cells were freeze-dried using a freeze dryer to obtain a lactic acid bacterium powder. The lactic acid bacterium powder was suspended at 1 mg/mL in PBS (PBS(-), pH 7.4, manufactured by Gibco/Thermo Fisher Scientific Inc.) into a lactic acid bacterium powder suspension.

**[Table 3]**

| | |
|---|---|
| Proteose Peptone No. 3 | 10.00 g |
| Beef extract | 10.00 g |
| Yeast extract | 5.00 g |
| D-Glucose | 20.00 g |
| Polysorbate 80 | 1.00 g |
| Ammonium citrate | 2.00 g |
| Sodium acetate | 5.00 g |
| Manganese sulfate | 0.10 g |
| Magnesium sulfate | 0.05 g |
| Dipotassium phosphate | 2.00 g |
| Distilled water | 1000 mL |

### <Evaluation of ability to activate follicle dermal papilla cell>

Each test sample (lactic acid bacterium powder suspension) thus obtained was evaluated for its follicle dermal papilla cell-activating effect. Specifically, human follicle dermal papilla cells were inoculated at a density of 1.2 × 10⁴ cells/well to a 96-well plate and cultured for 6 hours in a CO₂ incubator (5% CO₂, 37°C). Thereafter, 100 µL of D-MEM medium (containing 4% FBS) containing each test sample (final concentration: 1%) was added to each well. The plate thus supplemented was incubated for 72 hours in a CO₂ incubator (5% CO₂, 37°C). Then, the number of follicle dermal papilla cells in the culture solution was measured by absorptiometry. Cell Counting Kit-8 (manufactured by Dojindo Laboratories Co., Ltd.) was used in the measurement. The control used was PBS (PBS(-), pH 7.4, manufactured by Gibco/Thermo Fisher Scientific Inc.). The ability to activate follicle dermal papilla cells was evaluated on the basis of the ratio of follicle dermal papilla cell activation to that of the control defined as 1. The measurement results are shown in Table 4. As shown in Table 4, *Lactobacillus plantarum* was found to have high ability to activate follicle dermal papilla cells.

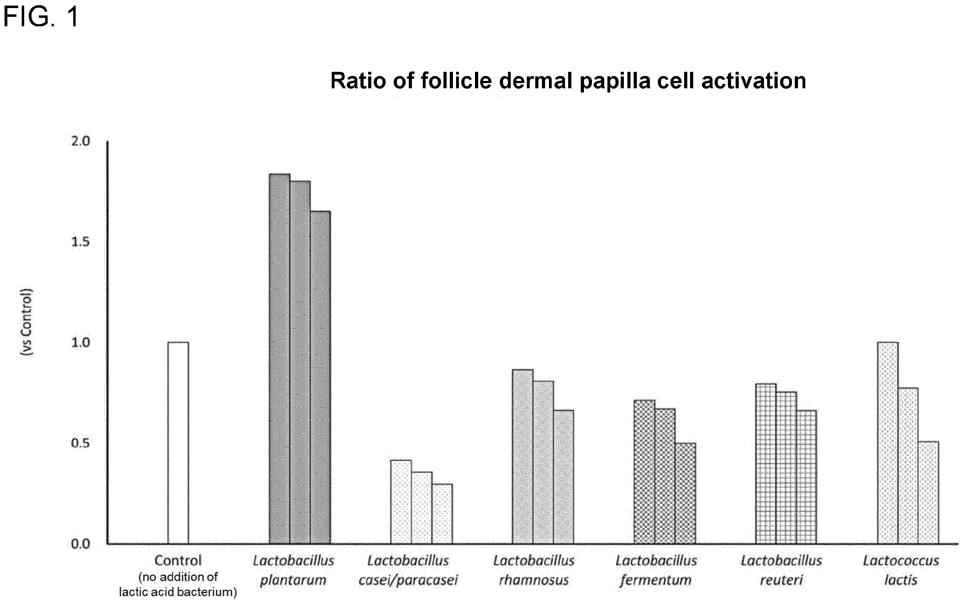

The test results are shown in Table 4. A graph of the results is shown in Figure 1.

### - Test results -

**[Table 4]**

| Bacterial species name | Bacterial strain name | Ratio of follicle dermal papilla cell activation (vs Control) |
|---|---|---|
| - | Control (no addition of lactic acid bacterium) | 1.000 |
| *Lactobacillus plantarum* | N793 | 1.836 |
| | N792 | 1.801 |
| | N807 | 1.651 |
| *Lactobacillus casei*/*paracasei* | N10 | 0.416 |
| | N32 | 0.357 |
| | N33 | 0.299 |
| *Lactobacillus rhamnosus* | N125 | 0.864 |
| | N128 | 0.808 |
| | N129 | 0.664 |
| *Lactobacillus fermentum* | N118 | 0.712 |
| | N218 | 0.669 |
| | Mill | 0.499 |
| *Lactobacillus reuteri* | N211 | 0.795 |
| | N210 | 0.755 |
| | N209 | 0.661 |
| *Lactobacillus lactis* | N819 | 1.000 |
| | N824 | 0.774 |
| | N826 | 0.507 |

As a result, *Lactobacillus plantarum* (3 species) was found to have high ability to activate follicle dermal papilla cells.

### 3. Intake test in clinical trial

The follicle dermal papilla cell activation test mentioned above demonstrated that *Lactobacillus plantarum* had high activity. The effectiveness of the N793 strain (NITE BP-03233) having the highest activity among the tested 3 species was verified in a clinical trial using a lactic acid bacterium-containing food product.

### Culture of lactic acid bacterium

The lactic acid bacterium was cultured at 37°C for 24 hours in MRS medium (manufactured by Becton, Dickinson and Company) shown in Table 3. Subsequently, the grown bacterial cells were harvested using a centrifuge. The harvested bacterial cells were washed with sterile water and harvested with the centrifuge. Washing and harvest were repetitively performed three times. After heat sterilization at 95°C for 15 minutes, the bacterial cells were harvested again with the centrifuge. The harvested bacterial cells were freeze-dried using a freeze dryer to obtain a lactic acid bacterium powder.

### Production of test food (lactic acid bacterium-containing food product) of Example

A lactic acid bacterium-containing food product was produced by blending the lactic acid bacterium powder and an excipient. The excipient contained hydrogenated maltose starch syrup, powder cellulose, calcium stearate, and a fine powder of silicon dioxide. The raw materials were mixed using a rocking mixer and thoroughly stirred. A tableting step involved sifting through a mesh and tableting in a tableting machine. The prepared lactic acid bacterium-containing food product had component composition shown in Table 5.

**[Table 5]**

| Test food (lactic acid bacterium-containing food product) | Amount blended (per tablet) |
|---|---|
| Lactic acid bacterium powder | 20 mg |
| Hydrogenated maltose starch syrup | 114 mg |
| Powder cellulose | 60 g |
| Calcium stearate | 3 mg |
| Fine powder of silicon dioxide | 3 mg |

Nutrient components (per tablet) of the lactic acid bacterium-containing food product are as shown in Table 6.

**[Table 6]**

| Nutrient component | Test food |
|---|---|
| Heat quantity | 0.76 kcal |
| Moisture | 0.00 g |
| Protein | 0.01 g |
| Lipid | 0.004 g |
| Carbohydrate | 0.17 g |

The production of the lactic acid bacterium-containing food product (tablet) was completed. A clinical trial was carried out using the tablet as a test food.

### Test subject

Test subjects were selected according to the following criteria 1) to 4).
1) Japanese males and females who were 20 years or older and 59 years or younger when their consents were obtained.
2) Persons who realized hair thinning.
3) Persons who were able to agree with no change in previously performed trimming manner (hair length, hair style, permanent, etc.), hair dying manner (hair dye) and hair washing manner (hair wash) during the test period.
4) Persons who were able to agree with local hair cut (treatment with an electric hair clipper with a length of approximately 0.5 mm left in a range of approximately 1 cm × 1 cm at approximately 3 cm on the right lateral side on the transverse line from the intersection between the median line of the head and a line (transverse line) connecting the uppermost parts of the right and left helixes) by a specialist.

The actual number of test subjects was 13 persons. Among them, the number of males was 6, and the number of females was 7. The average age was 50.23 ± 1.95 years.

### Eating method

An eating method for the test food mentioned above was performed as shown in Table 7.

**[Table 7]**

| Test food | Time zone | Timing | Amount used | Use method |
|---|---|---|---|---|
| lactic acid bacterium-containing food product | Morning | After breakfast | One tablet/time | Use one tablet together with water or lukewarm water within 30 minutes after breakfast. |
| | | | | If forgetting to use the test food, use it within the day. |
| | | | | Use the test food only once a day. If forgetting to use it within the day, do not carry it over to the next day. |

### Observation schedule of hair of head of test subject

The observation of hairs of the head of each test subject was carried out under the following schedule.

The number of times of observation was 6. The timing of observation was before use, 2 days after the start of use, 12 weeks after the start of use, 12 weeks + 2 days after the start of use, 24 weeks after the start of use, and 24 weeks + 2 days after the start of use. The intake duration of the test food at home was 168 days.

### Test results

For the observation of hairs of the head of each test subject (hair analysis), hairs in the local hair cut region (1 cm × 1 cm) mentioned above were examined using a digital microscope (HIROX Co., Ltd., KH-2000).

Examination such as hair analysis on the total number of hairs, the number of hairs, a hair thickness, the number of non-soft hairs, and hair loss as examination items was carried out. Statistical analysis was conducted by Dunnett's test after 12-week intake vs. before intake and after 24-week intake vs. before intake (+: P < 0.1, *: P < 0.05, **: P < 0.01, ***: P < 0.001). A significant trend was determined for +: P < 0.1, and a significant difference was determined for P < 0.05. The results about each item will be shown below.

### Hair analysis/total number of hairs

The total number of hairs was 163.7 ± 10.4 hairs before intake, 169.1 ± 10.6 hairs after 12-week intake, and 175.8 ± 11.3 hairs after 24-week intake. The total number of hairs compared with that before intake was increased by 5.4 hairs after 12-week intake and by 12.1 hairs after 24-week intake, confirming a significant difference after 24-week intake (P = 0.006). The results are shown in Table 8.

**[Table 8]**

| Item | Unit | n | Before intake | After 12-week intake | P value | After 24-week intake | P value |
|---|---|---|---|---|---|---|---|
| Total number of hairs | The number of hairs | 13 | 163.7±10.4 | 169.1±10.6 | P=0.266 | 175.8±11.3 | P=0.006 ** |

| | | | | | | | |
|---|---|---|---|---|---|---|---|
| Mean ± standard deviation, Dunnett (vs before intake) **: P<0.01 | | | | | | | |

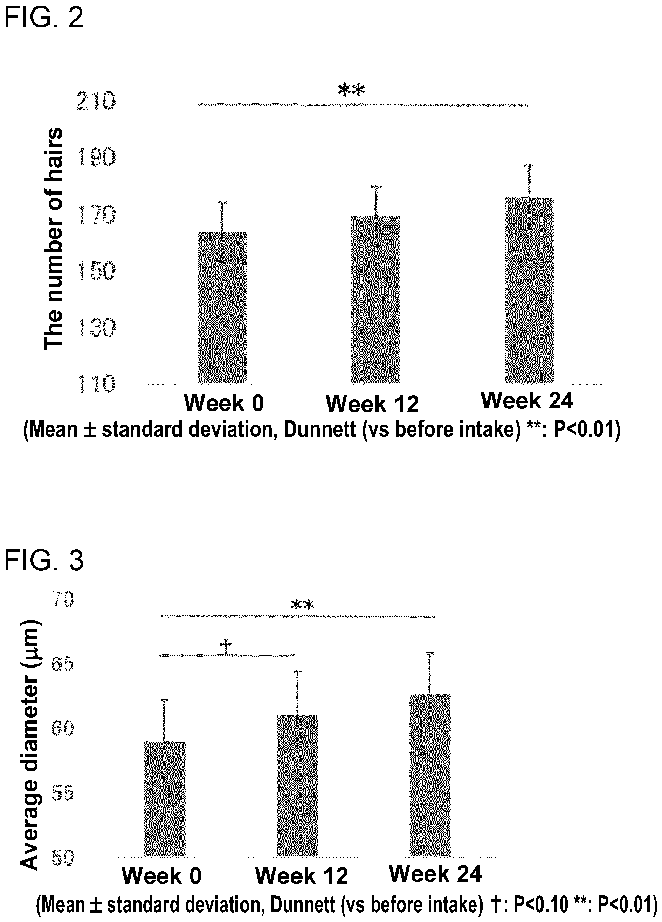

A graph thereof is shown in Figure 2.

### Hair analysis/hair thickness

The hair thickness was 58.9 ± 3.3 µm before intake, 61.0 ± 3.4 µm after 12-week intake, and 62.7 ± 3.1 µm after 24-week intake. The hair thickness compared with that before intake was increased by 2.1 µm after 12-week intake and by 3.8 µm after 24-week intake, confirming a significant trend after 12-week intake (P < 0.1) and also confirming a significant difference after 24-week intake (P = 0.001). The results are shown in Table 9.

**[Table 9]**

| Item | Unit | n | Before intake | After 12-week intake | P value | After 24-week intake | P value |
|---|---|---|---|---|---|---|---|
| Average diameter | µm | 13 | 58.9±3.3 | 61.0±3.4 | P=0.070 t | 62.7±3.1 | P=0.001 ** |

| | | | | | | | |
|---|---|---|---|---|---|---|---|
| Mean ± standard deviation, Dunnett (vs before intake) t: P<0.10 **: P<0.01 | | | | | | | |

A graph thereof is shown in Figure 3.

### Hair analysis/the number of non-soft hairs (strong hard hairs, hair diameter: 40 µm or larger)

The results about the number of strong hard hairs with a hair diameter of 40 µm or larger among hairs were 121.2 ± 12.3 hairs before intake, 131.8 ± 13.2 hairs after 12-week intake, and 141.0 ± 12.3 hairs after 24-week intake. The number of strong hard hairs compared with that before intake was significantly increased by 10.6 hairs (P = 0.025) after 12-week intake and by 19.8 hairs (P < 0.001) after 24-week intake. The results are shown in Table 10.

**[Table 10]**

| Item | Unit | n | Before intake | After 12-week intake | P value | After 24-week intake | P value |
|---|---|---|---|---|---|---|---|
| The number of non-soft hairs | The number of hairs | 13 | 121.2±12.3 | 131.8±13.2 | P=0.025 ^{∗} | 141.0±12.3 | P=0.001 ^{∗∗∗} |

| | | | | | | | |
|---|---|---|---|---|---|---|---|
| Mean ± standard deviation, Dunnett (vs before intake) ^{∗}: P<0.05 ^{∗∗∗}: P<0.001 | | | | | | | |

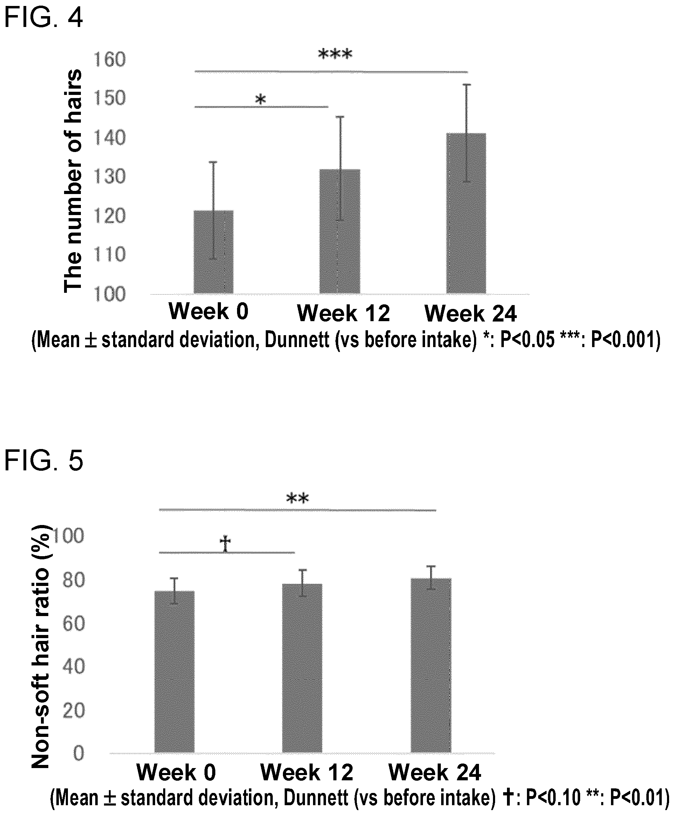

A graph thereof is shown in Figure 4.

### Hair analysis/non-soft hair ratio

The non-soft hair ratio (%) as to strong hard hairs with a hair diameter of 40 µm or larger among hairs was calculated as the number of non-soft hairs / the total number of hairs in the local cut region. The non-soft hair ratio was 74.8 ± 5.9% before intake, 78.3 ± 6.2% after 12-week intake, and 80.8 ± 5.4% after 24-week intake. The non-soft hair ratio compared with that before intake was increased by 3.5% after 12-week intake and by 6.0% after 24-week intake, confirming a significant trend after 12-week intake (P < 0.1) and also confirming a significant difference after 24-week intake (P = 0.002). The results are shown in Table 11.

**[Table 11]**

| Item | Unit | n | Before intake | After 12-week intake | P value | After 24-week intake | P value |
|---|---|---|---|---|---|---|---|
| Non-soft hair ratio | % | 13 | 74.8±5.9 | 78.3±6.2 | P=0.075 † | 80.8±5.4 | P=0.002 ^{∗∗} |

| | | | | | | | |
|---|---|---|---|---|---|---|---|
| Mean ± standard deviation, Dunnett (vs before intake) †: P<0.10 ^{∗∗}: P<0.01 | | | | | | | |

A graph thereof is shown in Figure 5.

### Hair analysis/the number of soft hairs

The results about the number of soft hairs with a hair diameter of less than 40 µm among hairs were 42.5 ± 11.6 hairs before intake, 37.2 ± 11.3 hairs after 12-week intake, and 34.8 ± 10.4 hairs after 24-week intake. The number of soft hairs compared with that before intake decreased by 5.3 hairs after 12-week intake and by 7.7 hairs after 24-week intake, confirming a significant difference after 24-week intake (P = 0.021). The results are shown in Table 12.

**[Table 12]**

| Item | Unit | n | Before intake | After 12-week intake | P value | After 24-week intake | P value |
|---|---|---|---|---|---|---|---|
| The number of soft hairs | The number of hairs | 13 | 42.5±11.6 | 37.2±11.3 | P=0.130 | 34.8±10.4 | P=0.021 * |

| | | | | | | | |
|---|---|---|---|---|---|---|---|
| Mean ± standard deviation, Dunnett (vs before intake) : P<0.05 | | | | | | | |

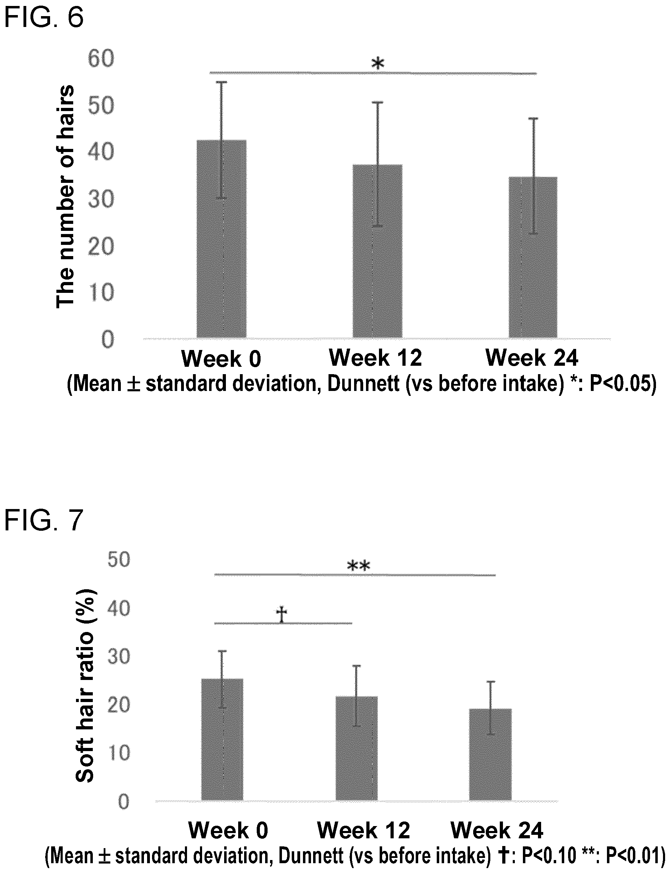

A graph thereof is shown in Figure 6.

### Hair analysis/soft hair ratio

The soft hair ratio (%) as to soft hairs with a hair diameter of less than 40 µm among hairs was calculated as the number of soft hairs / the total number of hairs in the local cut region. The soft hair ratio was 25.2 ± 5.9% before intake, 21.7 ± 6.2% after 12-week intake, and 19.2 ± 5.4% after 24-week intake. The soft hair ratio compared with that before intake decreased by 3.5% after 12-week intake and by 6.0% after 24-week intake, confirming a significant trend after 12-week intake (P < 0.1) and also confirming a significant difference after 24-week intake (P = 0.002). The results are shown in Table 13.

**[Table 13]**

| Item | Unit | n | Before intake | After 12-week intake | P value | After 24-week intake | P value |
|---|---|---|---|---|---|---|---|
| Soft hair ratio | % | 13 | 25.2±5.9 | 21.7±6.2 | P=0.075 † | 19.2±5.4 | P=0.002 ^{∗∗} |

| | | | | | | | |
|---|---|---|---|---|---|---|---|
| Mean ± standard deviation, Dunnett (vs before intake) t: P<0.10 **: P<0.01 | | | | | | | |

A graph thereof is shown in Figure 7.

### Hair analysis/hair loss

Fallen hairs were collected after hair washing and drying by a beautician, and the number of hairs was measured. The number of fallen hairs was 109.5 ± 15.4 hairs before intake, 78.6 ± 12.8 hairs after 12-week intake, and 75.9 ± 9.6 hairs after 24-week intake. The number of fallen hairs compared with that before intake exhibited a decrease trend by 30.9 hairs (P < 0.1) after 12-week intake and by 33.6 hairs (P < 0.1) after 24-week intake. The results are shown in Table 14.

**[Table 14]**

| Item | Unit | n | Before intake | After 12-week intake | P value | After 24-week intake | P value |
|---|---|---|---|---|---|---|---|
| The number of fallen hairs | The number of hairs | 13 | 109.5±15.4 | 78.6±12.8 | P=0.077 † | 75.9±9.6 | P=0.053 † |

| | | | | | | | |
|---|---|---|---|---|---|---|---|
| Mean ± standard deviation, Dunnett (vs before intake) t: P<0.10 | | | | | | | |

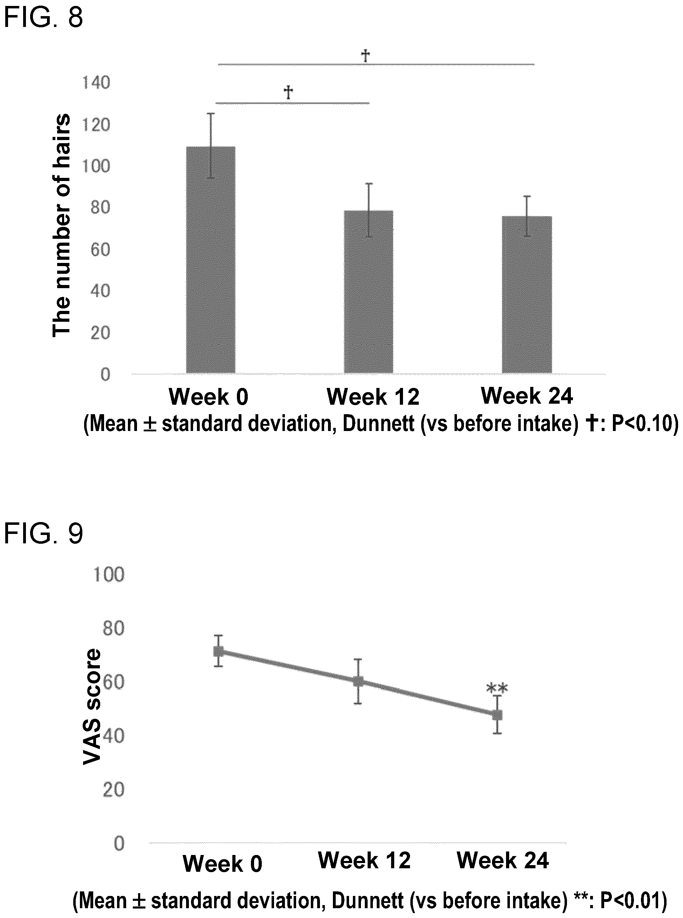

A graph thereof is shown in Figure 8.

### VAS questionnaire

Questionnaire based on the visual analogue scale method (VAS questionnaire) was carried out as to the perception of hair loss at the time of hair washing, the perception of hair loss at the time of hair styling, the realization of hair loss, hair thinness, and hair strength.

The questionnaire based on the visual analogue scale method (VAS questionnaire) involves setting extreme conditions to both ends, allowing a test subject himself or herself to express his or her subjective feeling upon entry as one vertical line on a written line segment of 100 mm in length, and measuring the length from the end to the vertical line as a VAS score. This method relates to the evaluation of test subjects' subjective feelings.

In this evaluation, for example, in the evaluation of the "perception of hair loss at the time of hair washing", the left end was set to the "feeling of no perception of hair loss (hairs at a drain, hairs attached to a towel after toweling off, etc.) at the time of hair washing" (score 0), and the right end was set to the "much worst feeling of the perception of hair loss (hairs at a drain, hairs attached to a towel after toweling off, etc.) at the time of hair washing" (score 100). Each test subject answered on the 100 mm line segment. Thus, a smaller number means better evaluation.

### 1) Perception of hair loss at time of hair washing (hairs at a drain, hairs attached to towel after toweling off, etc.)

The perception of hair loss was 71.5 ± 5.8 before intake, 60.2 ± 8.3 after 12-week intake, and 47.8 ± 7.0 after 24-week intake. The perception of hair loss compared with that before intake decreased by 11.3 after 12-week intake and by 23.7 after 24-week intake, confirming a significant difference after 24-week intake (P = 0.001). The results are shown in Table 15.

**[Table 15]**

| Item | Unit | n | Before intake | After 12-week intake | P value | After 24-week intake | P value |
|---|---|---|---|---|---|---|---|
| Hair loss at time of hair washing | - | 13 | 71.5±5.8 | 60.2±8.3 | P=0.123 | 47.8±7.0 | P=0.001 ^{∗∗} |

| | | | | | | | |
|---|---|---|---|---|---|---|---|
| Mean ± standard deviation, Dunnett (vs before intake) ^{∗∗}: P<0.01 | | | | | | | |

A graph thereof is shown in Figure 9.

### 2) Perception of hair loss at time of hair styling (hair drying, hair brushing, combing with hand, etc.)

The perception of hair loss was 65.5 ± 6.1 before intake, 53.5 ± 7.9 after 12-week intake, and 46.9 ± 7.4 after 24-week intake. The perception of hair loss compared with that before intake decreased by 12.0 after 12-week intake and by 18.6 after 24-week intake, confirming a significant trend after 12-week intake (P < 0.1) and also confirming a significant difference after 24-week intake (P = 0.007). The results are shown in Table 16.

**[Table 16]**

| Item | Unit | n | Before intake | After 12-week intake | P value | After 24-week intake | P value |
|---|---|---|---|---|---|---|---|
| Hair loss at time of hair styling | - | 13 | 65.5±6.1 | 53.5±7.9 | P=0.086 † | 46.9±7.4 | P=0.007 ^{∗∗} |

| | | | | | | | |
|---|---|---|---|---|---|---|---|
| Mean ± standard deviation, Dunnett (vs before intake) †: P<0.10 ^{∗∗}: P<0.01 | | | | | | | |

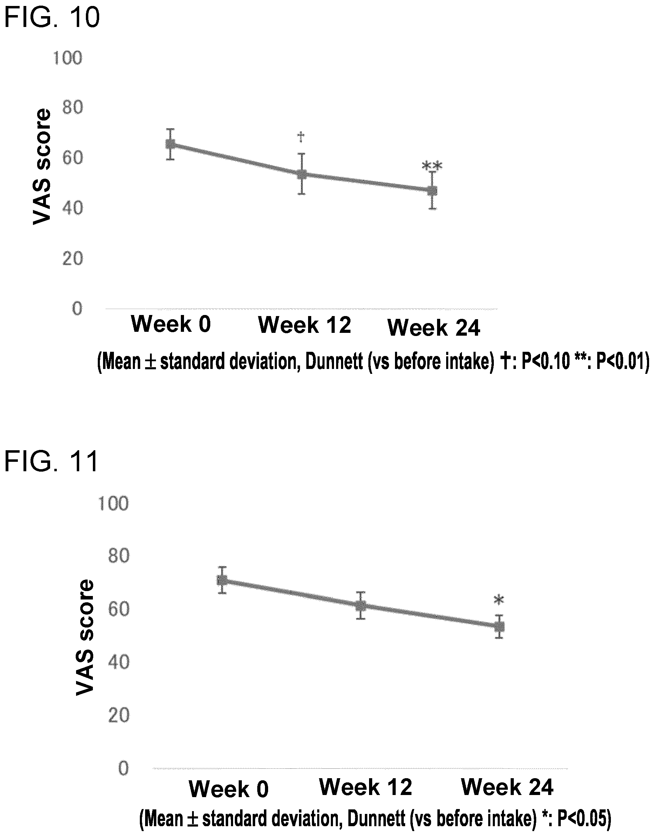

A graph thereof is shown in Figure 10.

### 3) Perception of lack of hair firmness or elasticity

The perception of a lack of hair firmness or elasticity was 70.8 ± 4.9 before intake, 61.3 ± 4.8 after 12-week intake, and 53.4 ± 4.3 after 24-week intake. The perception of a lack of hair firmness or elasticity compared with that before intake decreased by 9.5 after 12-week intake and by 17.4 after 24-week intake, confirming a significant difference after 24-week intake (P = 0.029). The results are shown in Table 17.

**[Table 17]**

| Item | Unit | n | Before intake | After 12-week intake | P value | After 24-week intake | P value |
|---|---|---|---|---|---|---|---|
| Lack of hair firmness or elasticity | - | 13 | 70.8±4.9 | 61.3±4.8 | P=0.284 | 53.4±4.3 | P=0.029 ^{∗} |

| | | | | | | | |
|---|---|---|---|---|---|---|---|
| Mean ± standard deviation, Dunnett (vs before intake) ^{∗}: P<0.05 | | | | | | | |

A graph thereof is shown in Figure 11.

### 4) Perception of hair thinness

The perception of hair thinness was 76.4 ± 4.8 before intake, 67.5 ± 6.2 after 12-week intake, and 57.2 ± 4.8 after 24-week intake. The perception of hair thinness compared with that before intake decreased by 8.9 after 12-week intake and by 19.2 after 24-week intake, confirming a significant difference after 24-week intake (P = 0.024). The results are shown in Table 18.

**[Table 18]**

| Item | Unit | n | Before intake | After 12-week intake | P value | After 24-week intake | P value |
|---|---|---|---|---|---|---|---|
| Hair thinness | - | 13 | 76.4±4.8 | 67.5±6.2 | P=0.374 | 57.2±4.8 | P=0.024 ^{∗} |

| | | | | | | | |
|---|---|---|---|---|---|---|---|
| Mean ± standard deviation, Dunnett (vs before intake) ^{∗}: P<0.05 | | | | | | | |

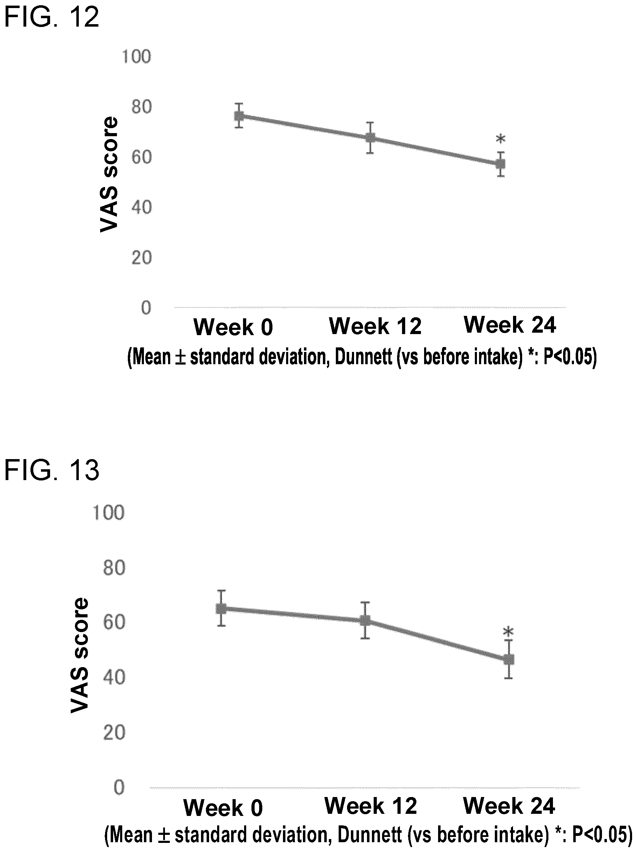

A graph thereof is shown in Figure 12.

### 5) Perception of lack of hair strength

The perception of a lack of hair strength was 65.0 ± 6.5 before intake, 60.6 ± 6.7 after 12-week intake, and 46.5 ± 6.9 after 24-week intake. The perception of a lack of hair strength compared with that before intake decreased by 4.4 after 12-week intake and by 18.5 after 24-week intake, confirming a significant difference after 24-week intake (P = 0.044). The results are shown in Table 19.

**[Table 19]**

| Item | Unit | n | Before intake | After 12-week intake | P value | After 24-week intake | P value |
|---|---|---|---|---|---|---|---|
| Lack of hair strength | - | 13 | 65.0±6.5 | 60.6±6.7 | P=0.794 | 46.5±6.9 | P=0.044 * |

| | | | | | | | |
|---|---|---|---|---|---|---|---|
| Mean ± standard deviation, Dunnett (vs before intake) : P<0.05 | | | | | | | |

A graph thereof is shown in Figure 13.

### 6) Perception of lack of hair volume

The perception of a lack of hair volume was 77.8 ± 5.6 before intake, 76.7 ± 5.0 after 12-week intake, and 59.3 ± 6.3 after 24-week intake. The perception of a lack of hair volume compared with that before intake decreased by 1.1 after 12-week intake and by 18.5 after 24-week intake, confirming a significant difference after 24-week intake (P = 0.022). The results are shown in Table 20.

**[Table 20]**

| Item | Unit | n | Before intake | After 12-week intake | P value | After 24-week intake | P value |
|---|---|---|---|---|---|---|---|
| Lack of hair volume | - | 13 | 77.8±5.6 | 76.7±5.0 | P=0.979 | 59.3±6.3 | P=0.022 ^{∗} |

| | | | | | | | |
|---|---|---|---|---|---|---|---|
| Mean ± standard deviation, Dunnett (vs before intake) ^{∗}: P<0.05 | | | | | | | |

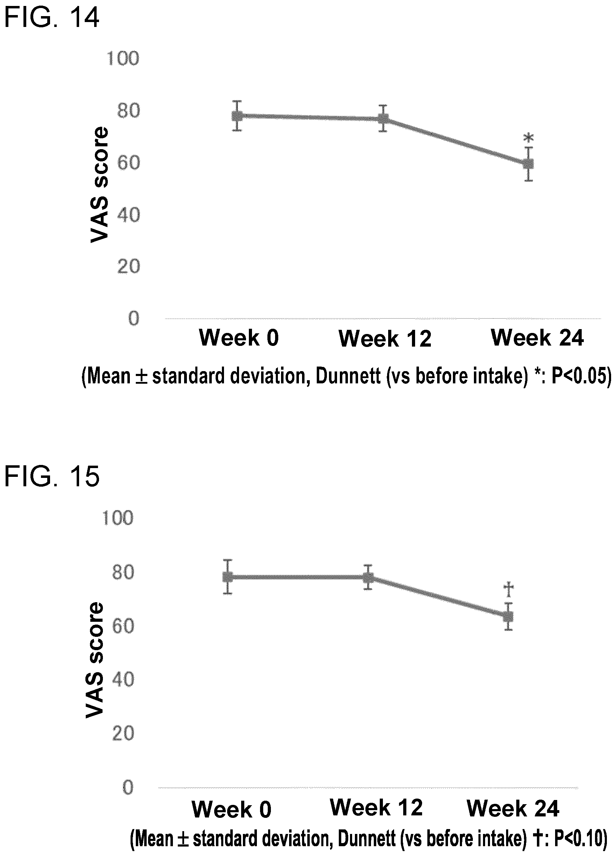

A graph thereof is shown in Figure 14.

### 7) Perception of noticeable hair sparseness at head skin (scalp)

The perception of noticeable hair sparseness was 78.0 ± 6.3 before intake, 77.8 ± 4.5 after 12-week intake, and 63.4 ± 4.9 after 24-week intake. The perception of noticeable hair sparseness compared with that before intake decreased by 0.2 after 12-week intake and by 14.6 after 24-week intake, confirming a significant trend after 24-week intake (P < 0.1). The results are shown in Table 21.

**[Table 21]**

| Item | Unit | n | Before intake | After 12-week intake | P value | After 24-week intake | P value |
|---|---|---|---|---|---|---|---|
| Noticeable hair sparseness at head skin (scalp) | - | 13 | 78.0±6.3 | 77.8±4.5 | P= 1.000 | 63.4±4.9 | P=0.057 † |

| | | | | | | | |
|---|---|---|---|---|---|---|---|
| Mean ± standard deviation, Dunnett (vs before intake) †: P<0.10 | | | | | | | |

A graph thereof is shown in Figure 15.

### 8) Perception of noticeable hair sparseness at top of head, hairline or parting

The perception of noticeable hair sparseness was 80.8 ± 5.0 before intake, 80.5 ± 4.5 after 12-week intake, and 63.5 ± 4.3 after 24-week intake. The perception of noticeable hair sparseness compared with that before intake decreased by 0.3 after 12-week intake and by 17.3 after 24-week intake, confirming a significant difference after 24-week intake (P = 0.013). The results are shown in Table 22.

**[Table 22]**

| Item | Unit | n | Before intake | After 12-week intake | P value | After 24-week intake | P value |
|---|---|---|---|---|---|---|---|
| Noticeable hair sparseness at top of head, hairline or parting | - | 13 | 80.8±5.0 | 80.5±4.5 | P=0.999 | 63.5±4.3 | P=0.013 ^{∗} |

| | | | | | | | |
|---|---|---|---|---|---|---|---|
| Mean ± standard deviation, Dunnett (vs before intake) ^{∗}: P<0.05 | | | | | | | |

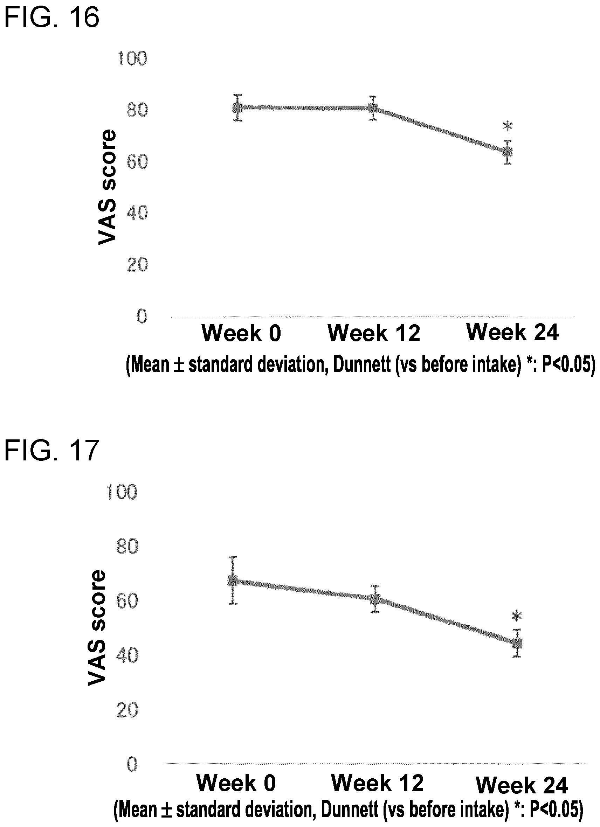

A graph thereof is shown in Figure 16.

### 9) Realization of extent of hair loss

The realization of the extent of hair loss was 67.3 ± 8.5 before intake, 60.5 ± 4.8 after 12-week intake, and 44.4 ± 4.8 after 24-week intake. The realization of the extent of hair loss compared with that before intake decreased by 6.8 after 12-week intake and by 22.9 after 24-week intake, confirming a significant difference after 24-week intake (P = 0.011). The results are shown in Table 23.

**[Table 23]**

| Item | Unit | n | Before intake | After 12-week intake | P value | After 24-week intake | P value |
|---|---|---|---|---|---|---|---|
| Realization of reduced extent of hair loss | - | 13 | 67.3±8.5 | 60.5±4.8 | P=0.581 | 44.4±4.8 | P=0.011 ^{∗} |

| | | | | | | | |
|---|---|---|---|---|---|---|---|
| Mean ± standard deviation, Dunnett (vs before intake) ^{∗}: P<0.05 | | | | | | | |

A graph thereof is shown in Figure 17.

### 4. Application test in clinical trial

The follicle dermal papilla cell activation test mentioned above demonstrated that *Lactobacillus plantarum* had high activity. The effectiveness of the N793 strain (NITE BP-03233) having the highest activity among the tested 3 species was verified in a clinical trial using a lactic acid bacterium-containing hair lotion.

### Culture of lactic acid bacterium

The lactic acid bacterium was cultured at 37°C for 24 hours in MRS medium (manufactured by Becton, Dickinson and Company) shown in Table 3. Subsequently, the grown bacterial cells were harvested using a centrifuge. The harvested bacterial cells were washed with sterile water and harvested with the centrifuge. Washing and harvest were repetitively performed three times. After heat sterilization at 95°C for 15 minutes, the bacterial cells were harvested again with the centrifuge. The harvested bacterial cells were freeze-dried using a freeze dryer to obtain a lactic acid bacterium powder.

### Production of lotion of Example

A lactic acid bacterium-containing hair lotion (type containing water or an alcohol as a main agent) was produced as a cosmetic product and a quasi-drug of the lactic acid bacterium powder and other components blended. The components used other than the lactic acid bacterium powder were water, ethanol, DPG (dipropylene glycol), Carbomer (carboxyvinyl polymer), calcium hydroxide, and PEG-hydrogenated castor oil.

More specifically, the lipid-soluble raw materials and the water-soluble raw materials were each dissolved by warming and then stirred (emulsified) using a vacuum emulsification apparatus to produce a lotion. The lactic acid bacterium powder was blended at 20 mg/3 mL.

In this way, the production of the lactic acid bacterium-containing hair lotion was completed. The following clinical trials were carried out using the hair lotion.

### Test subject

Test subjects were selected according to the following criteria 1) to 4).
1) Japanese males and females who were 20 years or older and 59 years or younger when their consents were obtained.
2) Persons who realized hair thinning.
3) Persons who were able to agree with no change in previously performed trimming manner (hair length, hair style, permanent, etc.), hair dying manner (hair dye) and hair washing manner (hair wash) during the test period.
4) Persons who were ablet to agree with local hair cut (treatment with an electric hair clipper with a length of approximately 0.5 mm left in a range of approximately 1 cm × 1 cm at approximately 3 cm on the right lateral side on the transverse line from the intersection between the median line of the head and a line (transverse line) connecting the uppermost parts of the right and left helixes) by a specialist.

The actual number of test subjects was 12 persons. Among them, the number of males was 7, and the number of females was 5. The average age was 48.83 ± 1.91 years.

### Use method

A method for using the test product mentioned above onto the scalp was performed as shown in Table 24.

**[Table 24]**

| Test product | Time zone | Timing | Amount used | Use method |
|---|---|---|---|---|
| Lactic acid bacterium-containing hair lotion | Morning or night | After hair washing or before bedtime | One marking (3 mL)/time | Use one marking (3 mL) within 30 minutes after hair washing in the morning. If not washing hairs in the morning or forgetting to use the test product, use it within 30 minutes after hair washing at night. |
| | | | | If also forgetting to use the test product at night, use it before bedtime. |
| | | | | Use the test product only once a day. If forgetting to use it within the day, do not carry it over to the next dav. |

### Observation schedule of hair of head of test subject

The observation of hairs of the head of each test subject was carried out under the following schedule.

The number of times of observation was 6. The timing of observation was before use, 2 days after the start of use, 12 weeks after the start of use, 12 weeks + 2 days after the start of use, 24 weeks after the start of use, and 24 weeks + 2 days after the start of use. The use duration of the lactic acid bacterium-containing hair lotion at home was 168 days.

### Test results

For the observation of hairs of the head of each test subject (hair analysis), hairs in the local hair cut region (1 cm × 1 cm) mentioned above were examined using a digital microscope (HIROX Co., Ltd., KH-2000).

Examination such as hair analysis on the total number of hairs, the number of hairs, a hair thickness, the number of non-soft hairs, and hair loss as examination items was carried out. Statistical analysis was conducted by Dunnett's test after 12-week use vs. before use and after 24-week use vs. before use (+: P < 0.1, *: P < 0.05, **: P < 0.01, ***: P < 0.001). A significant trend was determined for +: P < 0.1, and a significant difference was determined for P < 0.05. The results about each item will be shown below.

### Hair analysis/total number of hairs

The total number of hairs was 179.2 ± 6.9 hairs before use, 182.6 ± 7.8 hairs after 12-week use, and 193.3 ± 7.2 hairs after 24-week use. The total number of hairs compared with that before use was +3.4 after 12-week use and +14.1 after 24-week use, confirming a significant difference after 24-week use (P = 0.002). The results are shown in Table 25.

**[Table 25]**

| Item | Unit | n | Before use | After 12-week use | P value | After 24-week use | P value |
|---|---|---|---|---|---|---|---|
| Total number of hairs | The number of hairs | 12 | 179.2±6.9 | 182.6±7.8 | P=0.557 | 193.3±7.2 | P=0.002 ^{∗∗} |

| | | | | | | | |
|---|---|---|---|---|---|---|---|
| Mean ± standard deviation, Dunnett (vs before use) **: P<0.01 | | | | | | | |

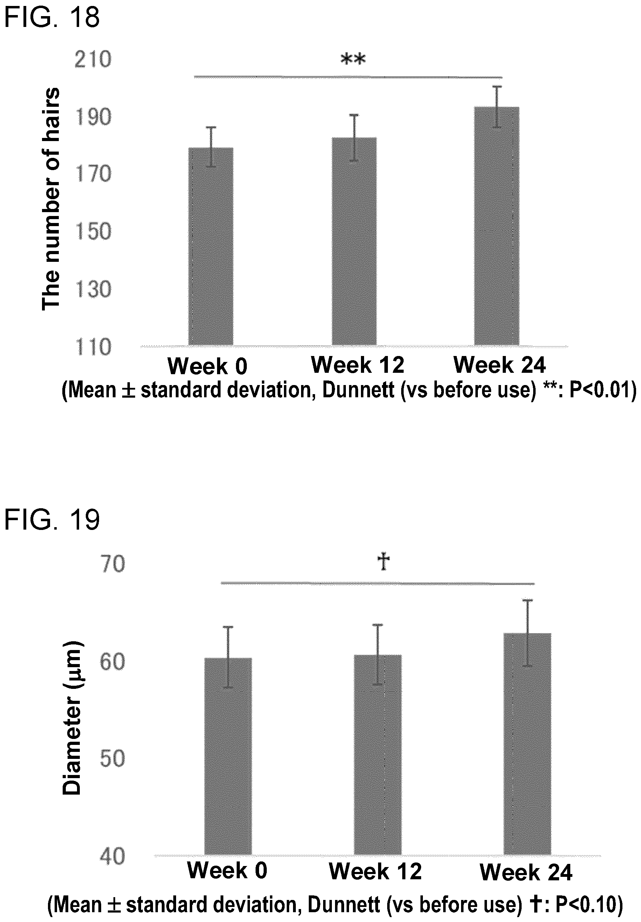

A graph thereof is shown in Figure 18.

### Hair analysis/hair thickness

The hair thickness was 60.4 ± 3.1 µm before use, 60.6 ± 3.1 µm after 12-week use, and 62.9 ± 3.4 µm after 24-week use. The hair thickness compared with that before use was +0.2 after 12-week use and +2.5 after 24-week use, confirming a significant trend after 24-week use (P = 0.051). The results are shown in Table 26.

**[Table 26]**

| Item | Unit | n | Before use | After 12-week use | P value | After 24-week use | P value |
|---|---|---|---|---|---|---|---|
| Average diameter | µm | 12 | 60.4±3.1 | 60.6±3.1 | P=0.962 | 62.9±3.4 | P=0.051 † |

| | | | | | | | |
|---|---|---|---|---|---|---|---|
| Mean ± standard deviation, Dunnett (vs before use) †: P<0.10 | | | | | | | |

A graph thereof is shown in Figure 19.

### Hair analysis/the number of non-soft hairs (strong hard hairs, hair diameter: 40 µm or larger)

The results about the number of strong hard hairs with a hair diameter of 40 µm or larger among hairs were 145.8 ± 8.8 hairs before use, 146.9 ± 8.6 hairs after 12-week use, and 162.1 ± 9.5 hairs after 24-week use. The number of strong hard hairs compared with that before use was +1.1 after 12-week use and +16.3 after 24-week use, confirming a significant difference after 24-week use (P < 0.001). The results are shown in Table 27.

**[Table 27]**

| Item | Unit | n | Before use | After 12-week use | P value | After 24-week use | P value |
|---|---|---|---|---|---|---|---|
| The number of non-soft hairs | The number of hairs | 12 | 145.8±8.8 | 146.9±8.6 | P=0.945 | 162.1±9.5 | P<0.001 *** |

| | | | | | | | |
|---|---|---|---|---|---|---|---|
| Mean ± standard deviation, Dunnett (vs before use) : P<0.001 | | | | | | | |

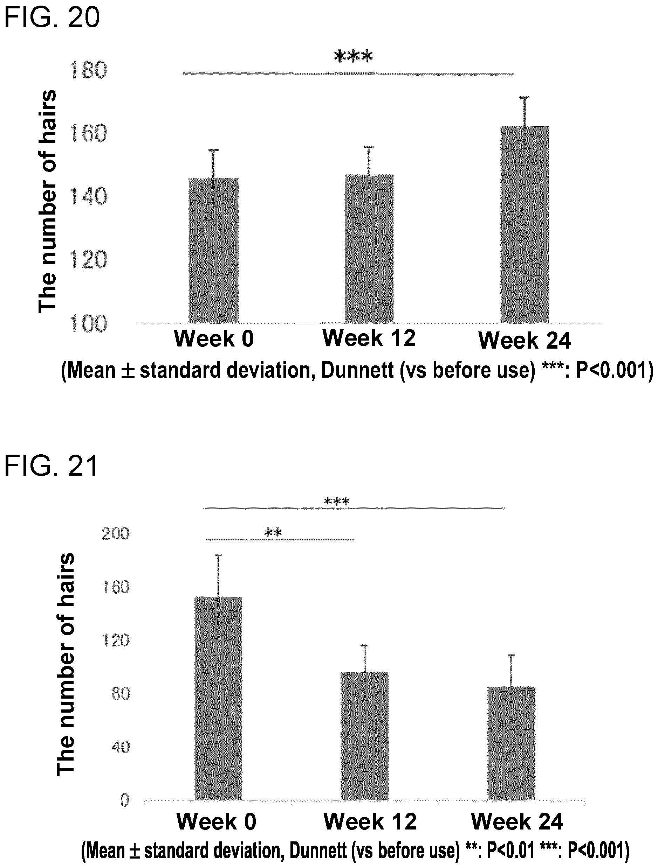

A graph thereof is shown in Figure 20.

### Hair analysis/hair loss

Fallen hairs were collected after hair washing and drying by a beautician, and the number of hairs was measured. The number of fallen hairs was 152.3 ± 31.5 hairs before use, 95.1 ± 20.4 hairs after 12-week use, and 84.5 ± 24.3 hairs after 24-week use. The number of fallen hairs compared with that before use was -57.2 after 12-week use and -67.8 after 24-week use, confirming a significant difference after 12-week use (P = 0.002) and after 24-week use (P < 0.001). The results are shown in Table 28.

**[Table 28]**

| Item | Unit | n | Before use | After 12-week use | P value | After 24-week use | P value |
|---|---|---|---|---|---|---|---|
| The number of fallen hairs | The number of hairs | 12 | 152.3±31.5 | 95.1±20.4 | P=0.002 ^{∗∗} | 84.5±24.3 | P<0.001 ^{∗∗∗} |

| | | | | | | | |
|---|---|---|---|---|---|---|---|
| Mean ± standard deviation, Dunnett (vs before use) ^{∗∗}: P<0.01 ^{∗∗∗}: P<0.001 | | | | | | | |

A graph thereof is shown in Figure 21.

### VAS questionnaire (lotion)

Questionnaire based on the visual analogue scale method (VAS questionnaire) was carried out as to the perception of hair loss at the time of hair washing, a lack of hair firmness or elasticity, hair thinness, hair strength (lack of hair strength), a lack of hair volume, noticeable hair sparseness at the head skin(scalp), noticeable hair sparseness at the top of the head, hairline or parting, and the realization of a reduced extent of hair loss.

The questionnaire based on the visual analogue scale method (VAS questionnaire) involves setting extreme conditions to both ends, allowing a test subject himself or herself to express his or her subjective feeling upon entry as one vertical line on a written line segment of 100 mm in length, and measuring the length from the end to the vertical line as a VAS score. This method relates to the evaluation of test subjects' subjective feelings.

In this evaluation, for example, in the evaluation of the "perception of hair loss at the time of hair washing", the left end was set to the "feeling of no perception of hair loss (hairs at a drain, hairs attached to a towel after toweling off, etc.) at the time of hair washing" (score 0), and the right end was set to the "much worst feeling of the perception of hair loss (hairs at a drain, hairs attached to a towel after toweling off, etc.) at the time of hair washing" (score 100). Each test subject answered on the 100 mm line segment. Thus, a smaller number means better evaluation.

### 1) Perception of hair loss (hairs at a drain, hairs attached to towel after toweling off, etc.) at time of hair washing

The perception of hair loss was 65.8 ± 7.8 before use, 55.0 ± 7.1 after 12-week use, and 50.7 ± 4.6 after 24-week use. The perception of hair loss compared with that before use was -10.8 after 12-week use and -15.1 after 24-week use, confirming a significant trend after 24-week use (P < 0.10). The results are shown in Table 29.

**[Table 29]**

| Item | Unit | n | Before use | After 12-week use | P value | After 24-week use | P value |
|---|---|---|---|---|---|---|---|
| Hair loss at time of hair washing | - | 12 | 65.8±7.8 | 55.0±7.1 | P=0.185 | 50.7±4.6 | P=0.051 † |

| | | | | | | | |
|---|---|---|---|---|---|---|---|
| Mean ± standard deviation, Dunnett (vs before use) t: P<0.10 | | | | | | | |

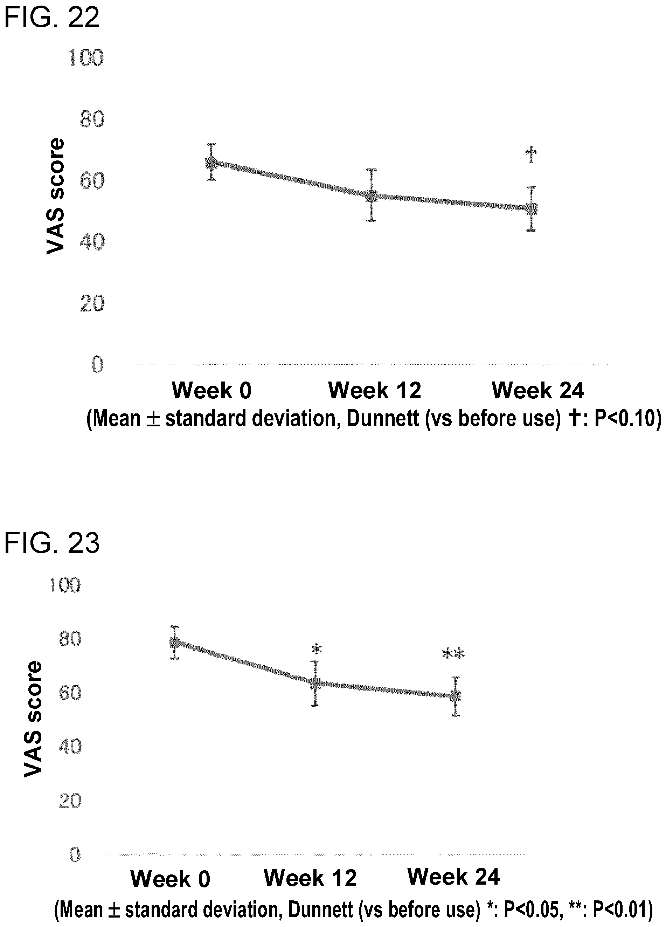

A graph thereof is shown in Figure 22.

### 2) Perception of lack of hair firmness or elasticity

The perception of a lack of hair firmness or elasticity was 78.5 ± 4.0 before use, 63.4 ± 4.5 after 12-week use, and 58.8 ± 6.2 after 24-week use. The perception of a lack of hair firmness or elasticity compared with that before use was -15.1 after 12-week use and -19.7 after 24-week use, confirming a significant difference after 12-week use (P = 0.015) and after 24-week use (P = 0.002). The results are shown in Table 30.

**[Table 30]**

| Item | Unit | n | Before use | After 12-week use | P value | After 24-week use | P value |
|---|---|---|---|---|---|---|---|
| Lack of hair firmness or elasticity | - | 12 | 78.5±4.0 | 63.4±4.5 | P=0.015 ^{∗} | 58.8±6.2 | P=0.002 ^{∗∗} |

| | | | | | | | |
|---|---|---|---|---|---|---|---|
| Mean ± standard deviation, Dunnett (vs before use) : P<0.05 : P<0.01 | | | | | | | |

A graph thereof is shown in Figure 23.

### 3) Perception of hair thinness

The perception of hair thinness was 78.0 ± 3.8 before use, 66.0 ± 5.3 after 12-week use, and 61.3 ± 5.8 after 24-week use. The perception of hair thinness compared with that before use was -12.0 after 12-week use and -16.7 after 24-week use, confirming a significant difference after 12-week use (P = 0.026) and after 24-week use (P = 0.002). The results are shown in Table 31.

**[Table 31]**

| Item | Unit | n | Before use | After 12-week use | P value | After 24-week use | P value |
|---|---|---|---|---|---|---|---|
| Hair thinness | - | 12 | 78.0±3.8 | 66.0±5.3 | P=0.026 ^{∗} | 61.3±5.8 | P=0.002 ^{∗∗} |

| | | | | | | | |
|---|---|---|---|---|---|---|---|
| Mean ± standard deviation, Dunnett (vs before use) ^{∗}: P<0.05 ^{∗∗}: P<0.01 | | | | | | | |

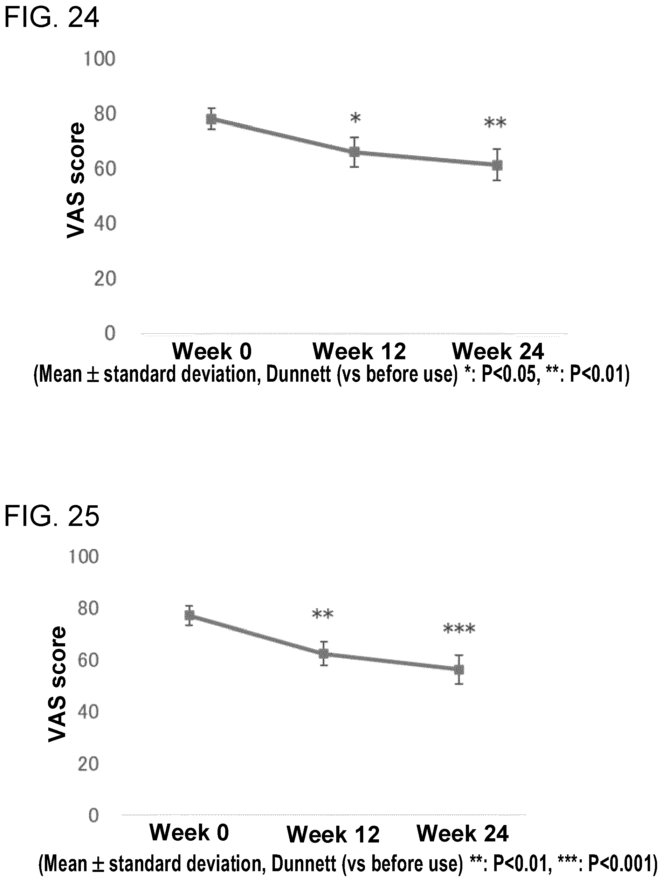

A graph thereof is shown in Figure 24.

### 4) Perception of lack of hair strength

The perception of a lack of hair strength was 77.1 ± 3.7 before use, 62.4 ± 4.6 after 12-week use, and 56.3 ± 5.5 after 24-week use. The perception of a lack of hair strength compared with that before use was -14.7 after 12-week use and - 20.8 after 24-week use, confirming a significant difference after 12-week use (P = 0.008) and after 24-week use (P < 0.001). The results are shown in Table 32.

**[Table 32]**

| Item | Unit | n | Before use | After 12-week use | P value | After 24-week use | P value |
|---|---|---|---|---|---|---|---|
| Lack of hair strength | - | 12 | 77.1±3.7 | 62.4±4.6 | P=0.008 ^{∗∗} | 56.3±5.5 | P<0.001 ^{∗∗∗} |

| | | | | | | | |
|---|---|---|---|---|---|---|---|
| Mean ± standard deviation, Dunnett (vs before use) ^{∗∗}: P<0.01 ^{∗∗∗}: P<0.001 | | | | | | | |

A graph thereof is shown in Figure 25.

### 5) Perception of lack of hair volume

The perception of a lack of hair volume was 83.3 ± 3.5 before use, 72.6 ± 6.5 after 12-week use, and 66.3 ± 4.9 after 24-week use. The perception of a lack of hair volume compared with that before use was -10.7 after 12-week use and -17.0 after 24-week use, confirming a significant difference after 24-week use (P = 0.006). The results are shown in Table 33.

**[Table 33]**

| Item | Unit | n | Before use | After 12-week use | P value | After 24-week use | P value |
|---|---|---|---|---|---|---|---|
| Lack of hair volume | - | 12 | 83.3±3.5 | 72.6±6.5 | P=0.083 | 66.3±4.9 | P=0.006 ^{∗∗} |

| | | | | | | | |
|---|---|---|---|---|---|---|---|
| Mean ± standard deviation, Dunnett (vs before use) ^{∗∗}: P<0.01 | | | | | | | |

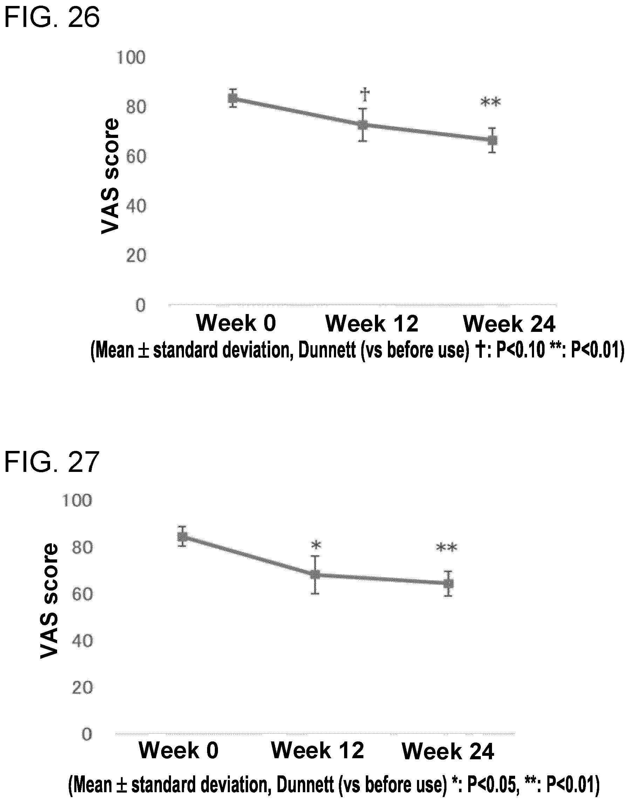

A graph thereof is shown in Figure 26.

### 6) Perception of noticeable hair sparseness at head skin (scalp)

The perception of noticeable hair sparseness was 84.6 ± 4.0 before use, 68.3 ± 8.0 after 12-week use, and 64.5 ± 5.3 after 24-week use. The perception of noticeable hair sparseness compared with that before use was -16.3 after 12-week use and -20.1 after 24-week use, confirming a significant difference after 12-week use (P = 0.024) and after 24-week use (P = 0.006). The results are shown in Table 34.

**[Table 34]**

| Item | Unit | n | Before use | After 12-week use | P value | After 24-week use | P value |
|---|---|---|---|---|---|---|---|
| Noticeable hair sparseness at head skin (scalp) | - | 12 | 84.6±4.0 | 68.3±8.0 | P=0.024 ^{∗} | 64.5±5.3 | P=0.006 ^{∗∗} |

| | | | | | | | |
|---|---|---|---|---|---|---|---|
| Mean ± standard deviation, Dunnett (vs before use) *: P<0.05 **:P<0.01 | | | | | | | |

A graph thereof is shown in Figure 27.

### 7) Perception of noticeable hair sparseness at top of head, hairline or parting

The perception of noticeable hair sparseness was 82.8 ± 4.2 before use, 69.3 ± 8.3 after 12-week use, and 65.8 ± 5.2 after 24-week use. The perception of noticeable hair sparseness compared with that before use was -13.5 after 12-week use and -17.0 after 24-week use, confirming a significant difference after 24-week use (P = 0.030). The results are shown in Table 35.

**[Table 35]**

| Item | Unit | n | Before use | After 12-week use | P value | After 24-week use | P value |
|---|---|---|---|---|---|---|---|
| Noticeable hair sparseness at top of head, hairline or parting | - | 12 | 82.8±4.2 | 69.3±8.3 | P=0.088 | 65.8±5.2 | P=0.030 ^{∗} |

| | | | | | | | |
|---|---|---|---|---|---|---|---|
| Mean ± standard deviation, Dunnett (vs before use) *: P<0.05 | | | | | | | |

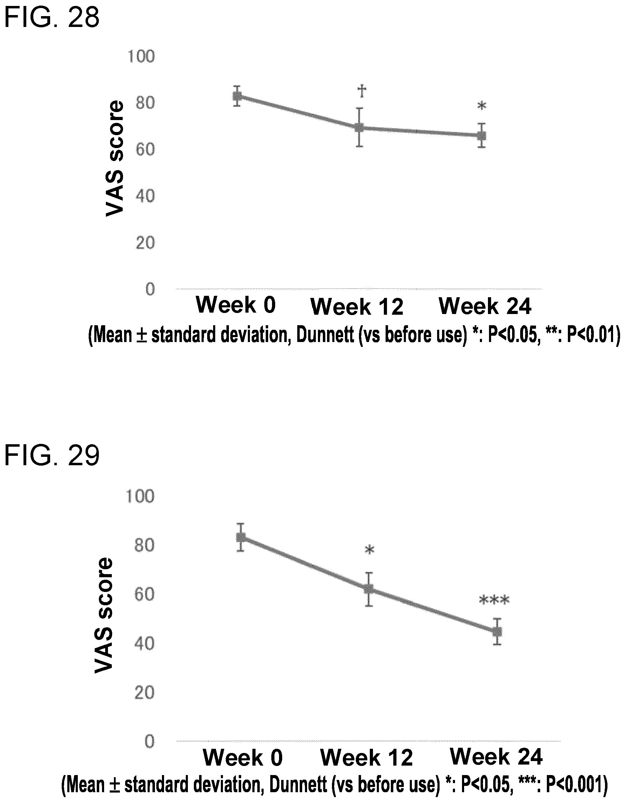

A graph thereof is shown in Figure 28.

### 8) Realization of reduced extent of hair loss

The realization of a reduced extent of hair loss was 83.1 ± 5.7 before use, 61.9 ± 6.7 after 12-week use, and 44.7 ± 5.3 after 24-week use. The realization of a reduced extent of hair loss compared with that before use was -21.2 after 12-week use and -38.4 after 24-week use, confirming a significant difference after 12-week use (P = 0.028) and after 24-week use (P < 0.001). The results are shown in Table 36.

**[Table 36]**

| Item | Unit | n | Before use | After 12-week use | P value | After 24-week use | P value |
|---|---|---|---|---|---|---|---|
| Realization of reduced extent of hair loss | - | 12 | 83.1±5.7 | 61.9±6.7 | P=0.028 ^{∗} | 44.7±5.3 | P<0.001 ^{∗∗∗} |

| | | | | | | | |
|---|---|---|---|---|---|---|---|
| Mean ± standard deviation, Dunnett (vs before use) ^{∗}: P<0.05 ^{∗∗∗}: P<0.001 | | | | | | | |

A graph thereof is shown in Figure 29.

The present application is based on Japanese Patent Application No. 2020-176970 filed on October 21, 2020 and Japanese Patent Application Nos. 2021-171280 and 2021-171281 filed on October 20, 2021, the contents of which are incorporated herein by reference in their entirety.

| | | |
|---|---|---|
| 0-1 | Form PCT/RO/134 | |
| | Indications Relating to Deposited Microorganism(s) or Other Biological Material (PCT Rule 13 bis) | |
| 0-1-1 | Prepared Using | JPO-PAS i430 |
| 0-2 | International Application No. | PCT/JP2021/038824 |
| 0-3 | Applicant's or agent's file reference | PNSH-00685WO |
| | | |
| 1 | The indications made below relate to the deposited microorganism(s) or other biological material referred to in the description on: | |
| 1-1 | paragraph | 0013 |
| 1-3 | Identification of deposit | |
| 1-3-1 | Name of depositary institution | NPMD NITE Patent Microorganisms Depositary (NPMD), Biological Resource Center, National Institute of Technology and Evaluation |
| 1-3-2 | Address of depositary institution | #122, 2-5-8 Kazusakamatari, Kisarazu-shi, Chiba 2920818, Japan |
| 1-3-3 | Date of deposit | July 31, 2020 (31.07 . 2020) |
| 1-3-4 | Accession Number | NPMD NITE BP-03233 |
| 1-5 | Designated States for Which Indications are Made | All designations |

| FOR RECEIVING OFFICE USE ONLY | | |
|---|---|---|
| 0-4 | This form was received with the international application: (ves or no) | yes |
| 0-4-1 | Authorized officer | Tsuneo Nagai |

| FOR INTERNATIONAL BUREAU USE ONLY | | |
|---|---|---|
| 0-5 | This form was received bv the international Bureau on: | |
| 0-5-1 | Authorized officer | |

## Claims

1. A food or drink product, a cosmetic product, a quasi-drug or a medicament comprising a lactic acid bacterium having a hair regrowth or hair growth effect in the human body, or a culture solution or a fermentation product thereof.

2. The food or drink product, the cosmetic product, the quasi-drug or the medicament comprising the lactic acid bacterium or the culture solution or the fermentation product thereof according to claim 1, wherein the food or drink product is any one selected from the group consisting of products named as refreshing sweets, a lactic acid bacterium-containing food product, a lactic acid bacteria beverage and a soft drink.

3. The food or drink product, the cosmetic product, the quasi-drug or the medicament comprising the lactic acid bacterium or the culture solution or the fermentation product thereof according to claim 1 or 2, wherein the food or drink product, the quasi-drug or the medicament is a tablet.

4. The food or drink product, the cosmetic product, the quasi-drug or the medicament according to any of claims 1 to 3, wherein the lactic acid bacterium or the culture solution or the fermentation product thereof belongs to a *Lactobacillus plantarum* species.

5. The food or drink product, the cosmetic product, the quasi-drug or the medicament according to any of claims 1 to 4, wherein the lactic acid bacterium or the culture solution or the fermentation product thereof is a lactic acid bacterium which is *a Lactobacillus plantarum* N793 strain (NITE BP-03233), or a culture solution or a fermentation product thereof.

6. The food or drink product, the cosmetic product, the quasi-drug or the medicament comprising the lactic acid bacterium or the culture solution or the fermentation product thereof according to claim 1, wherein the cosmetic product, the quasi-drug or the medicament is a liniment.

7. The food or drink product, the cosmetic product, the quasi-drug or the medicament according to claim 6, wherein the cosmetic product, the quasi-drug or the medicament is an ointment, a cream or a lotion.

8. The food or drink product, the cosmetic product, the quasi-drug or the medicament according to claim 6 or 7, wherein the lactic acid bacterium or the culture solution or the fermentation product thereof belongs to a *Lactobacillus plantarum* species.

9. The food or drink product, the cosmetic product, the quasi-drug or the medicament according to any of claims 6 to 8, wherein the lactic acid bacterium or the culture solution or the fermentation product thereof is a lactic acid bacterium which is *a Lactobacillus plantarum* N793 strain (NITE BP-03233), or a culture solution or a fermentation product thereof.
